# EUROPÄISCHE PATENTANMELDUNG

(11) **EP 1 719 504 A1**
(43) Veröffentlichungstag der Anmeldung: **08.11.2006**
(21) Anmeldenummer: 05009579.3
(22) Anmeldetag: 02.05.2005
(51) Int. Cl.: A61K 9/70, A61K 31/57

(54) **Festes transdermales therapeutisches System mit UV-Absorber**

(71) Anmelder: Schering AG, 13342 Berlin (DE)
(72) Erfinder: Langguth, Thomas, 07751 Jena (DE); Bracht, Stefan, 16548 Glienicke Nordbahn (DE)
(74) Vertreter: Cramer, Eva-Maria

(57) **Zusammenfassung**

Die Erfindung betrifft ein neues festes transdermales therapeutisches System mit UV-Absorber, besonders ausgelegt für lichtempfindliche pharmazeutische Wirkstoffe. Das UV stabile transdermales therapeutisches System (TTS) besteht aus einer Rückschicht 1, mindestens einer wirkstoffhaltigen Matrix 2 und einer abziehbaren Schutzfolie 3 , wobei wahlweise zwischen der Rückschicht 1 und der wirkstoffhaltigen Matrix 2 eine Klebeschicht 4 und eine Trennschicht 5 eingebracht sein. In der Rückschicht 1 oder in die wirkstoffhaltige Matrix 2 oder in die Klebeschicht 4 können UV-Absorber aus der Gruppe der Hydroxyphenyltriazine eingebettet sein.

Das erfindungsgemäße TTS realisiert eine hohe Stabilität mit einer niedrigen Konzentration an UV-Absorber. Damit kann insbesondere das Risiko für eine mögliche Hautirritation vermieden bzw. reduziert werden.

## Beschreibung

Die Erfindung betrifft ein festes transdermales therapeutisches System mit UV-Absorber. Das UV stabile transdermales therapeutisches System (TTS) ist besonders ausgelegt für lichtempfindliche pharmazeutische Wirkstoffe. Es besteht aus einer Rückschicht 1, mindestens einer wirkstoffhaltigen Matrix 2 und einer abziehbaren Schutzfolie 3 , wobei wahlweise zwischen der Rückschicht 1 und der wirkstoffhaltigen Matrix 2 eine Klebeschicht 4 und eine Trennschicht 5 eingebracht sein. In der Rückschicht 1 oder in die wirkstoffhaltige Matrix 2 oder in die Klebeschicht 4 können UV-Absorber aus der Gruppe der Hydroxyphenyltriazine eingebettet sein.
Es sind Versuche bekannt, lichtempfindliche Wirkstoffe, welche UV-A-und UV-B-Strahlen absorbieren, üblicherweise in Sonnencremes einzusetzen, wie von Briscart & Plaizier-Vercammen (Proc. 2^{nd} World Meeting on Pharmaceutics, Biopharmaceutics and Pharmaceutical Technology, APGI/ APV, 1998, 1231-1232) beschrieben.
Ferner ist aus der Patentliteratur bekannt, mit lichtempfindlichen Wirkstoffen versehene transdermale therapeutische Systeme (TTS) mittels optisch auffallenden aluminisierten oder lackierten Abdeckfolien als Rückschicht des TTS zu schützen.
Die WO-A1-00/56289 beschreibt ein Verfahren zum Schutz therapeutischer Zubereitungen, Systeme oder deren Bestandteile, wobei ein jeweils spezifischer Schutz gegen Abbau durch schädliche Faktoren, wie Luftsauerstoff, Wasser und/oder Licht realisiert werden soll. Es werden elektromagnetische Wellen absorbierende bzw. reflektierende Lichtschutzsubstanzen verwendet, wobei Absorptions- bzw. Reflexionsmittel eingesetzt werden, deren Absorptions- bzw. Reflexionsspektrum denjenigen Wellenlängenbereich umfasst, der für die Instabilität des lichtempfindlichen Stoffes bzw. seiner Bestandteile verantwortlich ist. Als Abdeckfolie werden hier u.a. eingefärbte Kunststofffolien verwendet, am Beispiel vom 1,4-Dihydopyridinderivat Lacidipin aufgezeigt.
Die Einfärbung von hochflexiblen Kunststofffolien erweist sich als schwierig und bietet durch häufig auftretende Risse in der Farbschicht der Kunststofffolie keinen zuverlässigen Lichtschutz.

Ferner sind aus WO-A2-02/34200 transdermale therapeutische Systeme (TTS) bekannt, die aus einer wirkstoffhaltigen Polymermatrix und einer Rückschicht bestehen, wobei Polymermatrix und Rückschicht fest verbunden sind bzw. ein Laminat bilden und sowohl die Polymermatrix als auch die Rückschicht eine im UV-Bereich absorbierende, farblose Substanz enthalten, die keine eigene pharmakologische Wirkung aufweist.
In der EP-A1-1452173 sind transdermale therapeutische Systeme aufgezeigt, welche aus einer Rückschicht, mindestens einer wirkstoffhaltigen Matrix und wahlweise einer Abziehfolie besteht sowie einen UV-Absorber enthält, wobei zwischen der Rückschicht und der der Hautoberfläche am weitesten abgewendeten wirkstoffhaltigen Matrix mindestens eine UV-Absorber enthaltende Klebschicht vorgesehen ist und zwischen der den UV-Absorber enthaltenden Klebschicht und der der Hautoberfläche am weitesten abgewendeten wirkstoffhaltigen Matrix mindestens eine Trennschicht enthalten ist, die wirkstoffundurchlässig und für den UV-Absorber undurchlässig ist. Als UV-Absorber werden hier die UV-Absorber aus der Gruppe p-Aminobenzoesäure, Aminobenzoesäurederivat, vorzugsweise 4-Dimethylaminobenzoesäure-2-ethylhexylester und/oder 4-Bis-(polyethoxyl)aminobenzoesäurepolyethoxyethylester, Zimtsäure,Zimtsäure-Derivate, vorzugsweise 4-Methoxylzimt-säureisoamylester und/oder 4-Methoxyzimtsäure-2-ethylhexyl-ester, 3-Benzylidenbornan-2-on, Benzylidenbornan-2-on-Derivate, vorzugsweise 3-(4')-Methylbenzylinden-bornan-2-on, 3-(4-Sulfon)benzylidenbornan-2-on und/oder 3-(4'-Trimethylammonium)benzylidenbornan-2-on-methylsulfat, Salicylsäurederivat, vorzugsweise 4-Isopropylbenzylsalicylat, Salicysäure-2-ethylhexylester, und/oder 3, 3, 5-Trimethyl-cyclohexylsalicylat, Benzotriazole, vorzugsweise 2-(5-chloro-2H-benzotriazole-2-yl)-6-(1,1-dimethylethyl)-4-methyl-phenol, 2, 4 ,6'-Trianilin-p-(carbo-2'-ethylhexyl-1'-oxy)-1, 3, 5-triazin, 3-Imidazol-4-yl-acrylsäure, 3-Imidazol-4-yl-.3-Imidazol-4-ylacrylsäure-Ester, 2-Phenylenbenzimidazol-5-sulfonsäure und/oder ihre K-, Na- und Triethanolamin (=TEA)-Salze, 2-Cyan-3,3-diphenylacrylsäure, Terephthaloyliden-di-campher-sulfonsäure, Butylmethoxy-dibenzoyl-methan, Benzophenon und/oder Benzophenon-Derivate, vorzugsweise Benzophenon-3 und/oder Benzophenon-4 ausgewählt sein kann/können.

Nachteilig an den bekannten Lösungen ist,
- dass die durch den zugesetzten UV-Absorber erzeugte Schutzwirkung für den Wirkstoff unvollständig ist,
- dass durch die unvollständige Schutzwirkung teilweise höhere Konzentrationen der UV-Absorber eingesetzt werden müssen, die sich negativ auf die Hautverträglichkeit der TTS auswirken können.

Aufgabe der Erfindung ist deshalb, eine mit einem lichtempfindlichen Wirkstoff versehene transdermal zu applizierende Arzneistoffzubereitung bereitzustellen, welche eine erhöhte Schutzwirkung für den Wirkstoff unter Verwendung einer möglichst geringen Konzentration des UV-Absorbers gewährleistet, die die vorher genannten Nachteile vermeidet.

Erfindungsgemäß wird die Aufgabe durch ein festes transdermales therapeutisches System mit UV-Absorber gelöst. Das UV stabile TTS besteht dabei in seiner Schichtfolge aus einer Rückschicht 1, mindestens einer wirkstoffhaltigen Matrix 2 und einer abziehbaren Schutzfolie 3 , wobei wahlweise zwischen der Rückschicht 1 und der wirkstoffhaltigen Matrix 2 eine Klebeschicht 4 und eine Trennschicht 5 eingebracht sein. In der Rückschicht 1 oder in die wirkstoffhaltige Matrix 2 oder in die Klebeschicht 4 können UV-Absorber aus der Gruppe der Hydroxyphenyltriazine eingebettet sein.

Erfindungsgemäß kann der UV-Absorber 2,4-bis-i[4-(2-ethyl-hexyloxy)-2-hydrody]-phenyly-6-(4-methoxyphenyl)-(1,3,5)-triazin sein.

Ferner kann erfindungsgemäß im festen transdermalen therapeutischen System das Flächengewicht der Matrix 2, 30 bis 150 g/m² betragen. Bevorzugt ist dabei ein Flächengewicht von 50 bis 120 g/m² und besonders bevorzugt von 100g/m².

Auch kann beim erfindungsgemäßen festen transdermalen therapeutischen System das Flächengewicht der Klebschicht 4, 5 bis 50 g/m². betragen. Bevorzugt ist dabei ein Flächengewicht von 20 bis 30 g/m².

Erfindungsgemäß kann der UV-Absorber in der Klebschicht 4 in der Konzentration von 0,5 bis 5 % (m/m) in gelöster form vorliegen. Bevorzugt ist dabei eine Konzentration von 1,0 bis 4,0 % und besonders bevorzugt von 1,5 bis 3,0 %.

Ferner kann erfindungsgemäß im festen transdermalen therapeutischen System die Matrix 2 und/ oder die Klebschicht 4 selbstklebend ausgeführt sein und im wesentlichen aus Polymeren bestehen, die aus den Gruppen Polyisobutylen, Polybuten,Polyacrylat, Polydimethylsiloxan, Styrol-Isopren-Blockpolymerisat oder Polyisopren ausgewählt sind.

Auch kann im erfindungsgemäßen festen transdermalen therapeutischen System die Trennschicht 5 eine Schichtdicke von 4 bis 23 µm aufweisen. Bevorzugt dabei ist die Schichtdicke von 4 bis 10 µm.

Erfindungsgemäß kann im festen transdermalen therapeutischen System Trennschicht 5 wirkstoffundurchlässig und für den UV-Absorber undurchlässig sein.

Ferner kann erfindungsgemäß im festen transdermalen therapeutischen System die Trennschicht 5 aus einem Barrierepolymer bestehen. Bevorzugt ist dabei Polyethylenterephthalat oder Polyacrylnitril oder Polyvinylchlorid oder Polyvinylidenchlorid oder dessen Copolymeren oder Kolaminaten.

Auch kann im erfindungsgemäßen festen transdermalen therapeutischen System die Rückschicht 1 wirkstoffdurchlässig sein und aus Polypropylen, Polyethylen, Polyurethan, Ethylen-Vinylacetat-Copolymer oder einem Mehrschichtverbund aus diesen Materialien untereinander oder mit anderen Materialien bestehen.

Erfindungsgemäß kann/können im festen transdermalen therapeutischen System der/die UV-Absorber farblos oder gelblich sein.

Ferner kann das erfindungsgemäße feste transdermale therapeutisches System transparent oder schwach opak ist.

Auch kann im erfindungsgemäßen festen transdermalen therapeutischen System als Wirkstoff mindestens ein Hormon wirksam werden.

Erfindungsgemäß kann der pharmazeutische Wirkstoffe ein Gestagen sein. Bevorzugt ist dabei Gestoden oder Levonorgestrel.

Ferner kann im erfindungsgemäßen festen transdermalen therapeutischen System zum Gestagen ein Estrogen zugefügt sein. Bevorzugt ist dabei Ethinylestradiol.

Auch kann erfindungsgemäß das feste transdermale therapeutische System ohne eine die Wirkstofffreisetzung steuernde Membran ausgerüstet sein.

Das erfindungsgemäße transdermale therapeutische System besitzt gegegenüber herkömmlichen Systemen mit lichtempfindlichen Wirkstoffgehalt folgende Vorteile:
- Die durch den UV-Absorber aus der Gruppe der Hydroxyphenyltriazine bereitgestellte Schutzwirkung ist verstärkt ist und
- die für die Erreichung einer Schutzwirkung notwendige Konzentration des UV-Absorbers aus der Gruppe der Hydroxyphenyltriazine ist reduziert.
- Damit kann insbesondere das Risiko für eine mögliche Hautirritation vermieden bzw. reduziert werden.

Die Erfindung wird durch nachfolgende Beispiele näher erläutert.

### Beispiel 1

Es wurden zwei Formulierungen eines lichtempfindlichen Wirkstoffes aus der Gruppe der Gestagene hergestellt. Formulierung I enthält eine Klebeschicht 4 und eine Trennschicht 5, wobei die Klebeschicht 43Gew.% einer UV-absorbierende Substanz enthält. Formulierung II enthält keine Klebeschicht 4 und Trennschicht 5 und dient als Vergleichsformulierung. Beide Formulierungen enthalten eine wirkstoffhaltige Matrix 2 mit einem lichtempfindlichem Gestagen und wurden mit einer Rückschicht1 aus Polyethylen ausgestattet, wodurch jeweils ein TTS erhalten wird.
Die Formulierung I hat folgende Zusammensetzung:
1. wirkstoffhaltige Matrix:
   - 1,9 % Gestagen
   - 98,1 % Polyisobutylen basierendes Klebemittel
2. Klebeschicht:
   - 3 % Tinuvin® 326
   - 97 % Polyisobutylen basierendes Klebemittel
   Tinuvin®326 (Fa. CIBA, Lampertheim) ist ein UV-Absorber der Hydroxyphenylbenzotriazol-Klasse.
   Zur Untersuchung der Lichtschutzwirkung wurden beide Formulierungen mit Licht mit einem UV-Spektrum von 300 - 800 nm über eine Dauer von bis zu 14h bestrahlt. Als Strahlungsquelle wurde eine Xenon-Lampe verwendet. Zwischen der Strahlungsquelle und den zu bestrahlenden Proben wurde eine Filtersystem (Typ: Suprax®-Filter) gesetzt, um die Bestrahlung unter realistischen Anwendungsbedingungen der TTS nachzuahmen. Anschließend wurde der Wirkstoffgehalt in den TTS bestimmt.

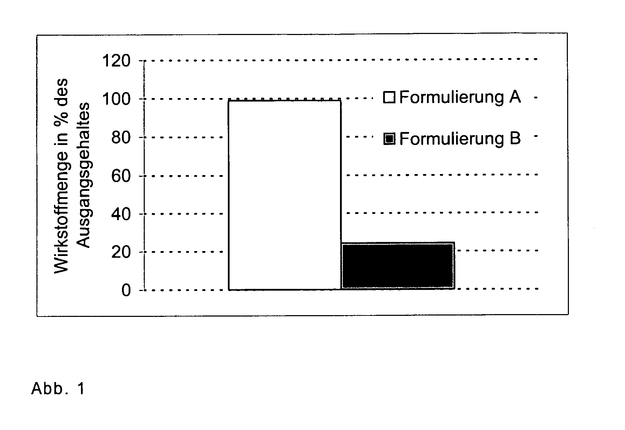

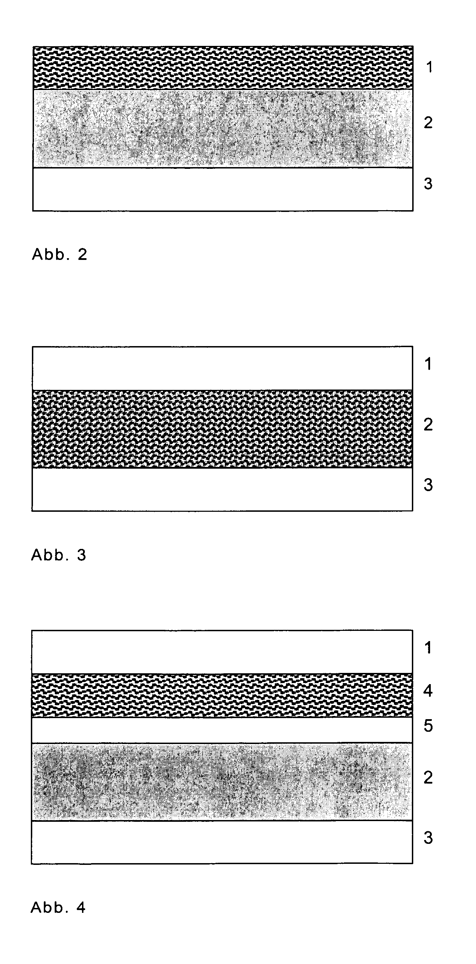

   Abbildung 1 zeigt auf, dass die TTS der Formulierung A, die eine Klebeschicht mit UV-absorbierender Substanz und eine Trennschicht enthielt, nach Bestrahlung über 14 h noch ca. 99% der ursprünglich eingesetzten Menge des lichtempfindlichen Wirkstoffes enthielt, wogegen die TTS der Formulierung B bereits nach der Bestrahlung von 7h nur noch ca. 24 % der ursprünglich eingesetzten Menge des lichtempfindlichen Wirkstoffs enthielt.
   Es kann der Nachweis geführt werden, dass das erfindungsgemäße System unter realistischen Anwendungsbedingungen einen verbesserten Sonnenschutz aufweist, da die UV-Schutzwirkung des erfindungsgemäßen Systems (Formulierung A) wesentlich höher als die des Vergleichssystems (Formulierung B) war.

### Beispiel 2

Formulierung mit einem lichtempfindlichen Wirkstoffes aus der Gruppe der Gestagene mit jeweils einer Klebschicht und Trennschicht, bei dem die Trennschichte aus Polyethylenterephthalat (Hostaphan®, Fa. Mitsubishi Polyester, Wiesbaden) besteht.
Die Formulierung hat folgende Zusammensetzung:
1. wirkstoffhaltige Matrix:
   - 1,9 % Gestagen
   - 98,1 % Polyisobutylen basierendes Klebemittel
2. Klebeschicht 1 und 2:
   - 3 % Uvinul®MC80
   - 97 % Polyacrylat basierendes Klebemittel
      Uvinul®MC 80 (Fa. BASF, Ludwigshafen) ist ein Methoxyzimtsäure-Derivat.

### Beispiel 3

Formulierung mit einem lichtempfindlichen Wirkstoffes aus der Gruppe der Gestagene mit jeweils zwei Klebschichten und Trennschichten, bei dem die Trennschichten aus Polyethylenterephthalat (Hostaphan®, Fa. Mitsubishi Polyester, Wiesbaden) bestehen.
Die Formulierung I hat folgende Zusammensetzung:
3. wirkstoffhaltige Matrix:
   - 1,9 % Gestagen
   - 98,1 % Polyisobutylen basierendes Klebemittel
4. Klebeschicht 1 und 2:
   - 3 % Uvinul®M40
   - 97 % Polyacrylat basierendes Klebemittel
      Uvinul®M40 (Fa. BASF, Ludwigshafen) ist ein Benzophenon-Derivat.

### Beispiel 4 bis 12

Formulierung mit einem lichtempfindlichen Wirkstoffes aus der Gruppe der Gestagene mit jeweils mindestens einer Klebschicht und Trennschicht, bei denen die wirkstoffhaltige Matrix analog zu den Beispielen 1 bis 3 ausgeführt ist und die Klebschicht eine Polyisobutylen basierendes Klebemittel enthält und die nachfolgend genannten Zusammensetzungen besitzt.

| Zusammensetzung der Klebschicht | Beispiel 4 | Beispiel 5 | Beispiel 6 | Beispiel 7 | Beispiel 8 | Beispiel 9 | Beispiel 10 | Beispiel 11 | Beispiel 12 |
|---|---|---|---|---|---|---|---|---|---|
| Tinuvin®326 [%] | 2 | 2 | 2 | 3 | 3 | 3 | 4 | 4 | 4 |
| Polyisobutylen basierendes Klebemittel [%] | 98 | 98 | 98 | 97 | 97 | 97 | 96 | 96 | 96 |
| Flächengewicht [g/m²] | 20 | 30 | 50 | 20 | 30 | 50 | 20 | 30 | 50 |

### Beispiel 13 bis 21

Formulierung mit einem lichtempfindlichen Wirkstoffes aus der Gruppe der Gestagene mit jeweils mindestens einer Klebschicht und Trennschicht, bei denen die wirkstoffhaltige Matrix analog zu den Beispielen 1 bis 3 ausgeführt ist und die Klebschicht eine Polyacrylat basierendes Klebemittel enthält und die nachfolgend genannten Zusammensetzungen besitzt.

| Zusammensetzung der Klebschicht | Beispiel 13 | Beispiel 14 | Beispiel 15 | Beispiel 16 | Beispiel 17 | Beispiel 18 | Beispiel 19 | Beispiel 20 | Beispiel 21 |
|---|---|---|---|---|---|---|---|---|---|
| Tinuvin®326 [%] | 2 | 2 | 2 | 3 | 3 | 3 | 4 | 4 | 4 |
| Polyacryralat basierendes Klebemittel [%] | 98 | 98 | 98 | 97 | 97 | 97 | 96 | 96 | 96 |
| Flächengewicht [^{g}/m²] | 20 | 30 | 50 | 20 | 30 | 50 | 20 | 30 | 50 |

### Beispiel 22 bis 30

Formulierung mit einem lichtempfindlichen Wirkstoffes aus der Gruppe der Gestagene mit jeweils mindestens einer Klebschicht und Trennschicht, bei denen die wirkstoffhaltige Matrix analog zu den Beispielen 1 bis 3 ausgeführt ist und die Klebschicht die nachfolgend genannten Zusammensetzungen besitzt.

| Zusammensetzung der Klebschicht | Beispiel 22 | Beispiel 23 | Beispiel 24 | Beispiel 25 | Beispiel 26 | Beispiel 27 | Beispiel 28 | Beispiel 29 | Beispiel 30 |
|---|---|---|---|---|---|---|---|---|---|
| Uvinol ®MC80 | 2 | 5 | 8 | - | - | - | - | - | - |
| Uvinol ®M40 | - | - | - | 2 | 5 | 8 | 2 | 5 | 8 |
| Polyisobutylen basierendes Klebemittel [%] | 98 | 95 | 92 | 98 | 95 | 92 | - | - | - |
| Flächengewicht [g/m²] | 20 | 30 | 50 | 20 | 30 | 50 | 20 | 30 | 50 |

## Patentansprüche

1. Festes transdermales therapeutisches System mit UV-Absorber, dessen Schichtenfolge aus mindestens drei Schichten besteht, wobei diese Schichtenfolge am weitesten von der Haut entfernt beginnt mit einer Rückschicht (1), einer mindestens einschichtigen wirkstoffhaltigen Matrix (2), einer abziehbaren Schutzfolie (3) und wahlweise zwischen Rückschicht (1) und wirkstoffhaltiger Matrix (2) eingebrachten Klebeschicht (4) und zu wirkstoffhaltigen Matrix (2) hin folgenden Trennschicht (5), **dadurch gekennzeichnet, dass** de/die UV-Absorber aus der Gruppe der Hydroxyphenyltriazine eingebettet ist/sind in die Rückschicht (1) oder in die wirkstoffhaltige Matrix (2) oder in die Klebeschicht (4).

2. Festes transdermales therapeutisches System nach Anspruch 1, **dadurch gekennzeichnet, dass** der UV-Absorber aus der Gruppe der Hydroxyphenyltriazine 2,4-bis-i[4-(2-ethyl-hexyloxy)-2-hydrody]-phenyly-6-(4-methoxyphenyl)-(1,3,5)-triazin ist.

3. Festes transdermales therapeutisches System nach mindestens einem der Ansprüche 1 und 2 , **dadurch gekennzeichnet, dass** das Flächengewicht der Matrix (2) 30 bis 150 g/m², vorzugsweise 50 bis 120 g/m² beträgt.

4. Festes transdermales therapeutisches System nach mindestens einem der vorhergehenden Ansprüche, **dadurch gekennzeichnet, dass** das Flächengewicht der Klebschicht (4) 5 bis 50 g/m², vorzugsweise 20 bis 30 g/m² beträgt.

5. Festes transdermales therapeutisches System nach Anspruch 4, **dadurch gekennzeichnet, dass** in der Klebschicht (4) mindestens ein UV-Absorber in einer Konzentration von 0,5 bis 5 % (m/m), vorzugsweise 1,0 bis 4,0 % (m/m) in gelöster Form vorliegt.

6. Festes transdermales therapeutisches System nach mindestens einem der vorhergehenden Ansprüche , **dadurch gekennzeichnet, dass** die Matrix (2) und/ oder die Klebschicht (4) selbstklebend ausgeführt ist/ sind und im wesentlichen aus Polymeren besteht, die aus den Gruppen Polyisobutylen, Polybuten,Polyacrylat, Polydimethylsiloxan, Styrol-Isopren-Blockpolymerisat oder Polyisopren ausgewählt sind.

7. Festes transdermales therapeutisches System nach mindestens einem der vorhergehenden Ansprüche, **dadurch gekennzeichnet, dass** die Trennschicht (5) eine Schichtdicke von 4 bis 23 µm, vorzugsweise von 4 bis 10 µm, aufweist.

8. Festes transdermales therapeutisches System nach Anspruch 7, **dadurch gekennzeichnet, dass** die Trennschicht (5) wirkstoffundurchlässig und für den UV-Absorber undurchlässig ist.

9. Festes transdermales therapeutisches System nach mindestens einem der vorhergehenden Ansprüche , **dadurch gekennzeichnet, dass** die Trennschicht (5) aus einem Barrierepolymer besteht, vorzugsweise aus Polyethylenterephthalat, Polyacrylnitril, Polyvinylchlorid, Polyvinylidenchlorid oder dessen Copolymeren oder Kolaminaten.

10. Festes transdermales therapeutisches System nach mindestens einem der vorhergehenden Ansprüche , **dadurch gekennzeichnet, dass** die Rückschicht (1) wirkstoffdurchlässig ist und vorzugsweise aus Polypropylen, Polyethylen, Polyurethan, Ethylen-Vinylacetat-Copolymer oder einem Mehrschichtverbund aus diesen Materialien untereinander oder mit anderen Materialien besteht.

11. Festes transdermales therapeutisches System nach mindestens einem der vorhergehenden Ansprüche , **dadurch gekennzeichnet, dass** der UV-Absorber farblos oder gelblich ist.

12. Festes transdermales therapeutisches System nach mindestens einem der vorhergehenden Ansprüche 1 bis 10, **dadurch gekennzeichnet, dass** das transdermale therapeutische System transparent oder schwach opak ist.

13. Festes transdermales therapeutisches System nach mindestens einem der vorhergehenden Ansprüche, **dadurch gekennzeichnet, dass** als Wirkstoff mindestens ein Hormon wirksam wird.

14. Festes transdermales therapeutisches System nach Anspruch 14 **dadurch gekennzeichnet, dass** der pharmazeutische Wirkstoff Gestagen, vorzugsweise Gestoden oder Levonorgestrel, ist.

15. Festes transdermales therapeutisches System nach den Ansprüchen 13 und 14, **dadurch gekennzeichnet, dass** zum Gestagen, vorzugsweise Gestoden, ein Estrogen, vorzugsweise Ethinylestradiol zugefügt ist.

16. Festes transdermales therapeutisches System nach mindestens einem der vorhergehenden Ansprüche, **dadurch gekennzeichnet, dass** das transdermale therapeutische System ohne eine die Wirkstofffreisetzung steuernde Membran ausgerüstet ist.
